(19) Europäisches Patentamt
European Patent Office
Office européen des brevets

(11) Numéro de publication: **0 089 886**
**B1**

(12) **FASCICULE DE BREVET EUROPEEN**

(45) Date de publication du fascicule du brevet: **12.10.88**

(51) Int. Cl.⁴: **C 07 B 57/00,** C 07 B 55/00, C 07 C 101/00, C 07 D 213/48

(21) Numéro de dépôt: **83400546.4**

(22) Date de dépôt: **16.03.83**

(54) **Procédé de préparation d'un alpha-amino-acide libre L.**

(30) Priorité: **23.03.82 FR 8204886**

(43) Date de publication de la demande:
**28.09.83 Bulletin 83/39**

(45) Mention de la délivrance du brevet:
**12.10.88 Bulletin 88/41**

(84) Etats contractants désignés:
**AT BE CH DE GB IT LI LU NL SE**

(56) Documents cités:
**EP-A-0 057 092**
**DE-A-2 215 853**
**GB-A-2 000 496**
**CHEMICAL ABSTRACTS, vol. 75, no. 25, 20 décembre 1971, page 24, no. 147947w, Columbus, Ohio, USA**
**CHEMICAL ABSTRACTS, vol. 95, no. 1, 6 juillet 1981, page 736, no. 7722g, Columbus, Ohio, USA**
**CHEMICAL ABSTRACTS, vol. 89, no. 21, 20 novembre 1978, page 642, no. 180323b, Columbus, Ohio, USA**

(73) Titulaire: **CENTRE NATIONAL DE LA RECHERCHE SCIENTIFIQUE (CNRS)**
**15, Quai Anatole France**
**F-75007 Paris (FR)**
(73) Titulaire: **INSTITUT NATIONAL DE LA SANTE ET DE LA RECHERCHE MEDICALE (INSERM)**
**101, rue de Tolbiac**
**F-75654 Paris Cédex 13 (FR)**

(72) Inventeur: **Commeyras, Auguste**
**Impasse des Ecoles Clapiers**
**F-34170 Castelnau le Lez (FR)**
Inventeur: **Previero, Aldo**
**Mas de la Droletta rue du Père Prevost**
**F-34000 Montpellier (FR)**
Inventeur: **Pugniere, Martine**
**5 rue du Canal**
**F-34000 Montpellier (FR)**

(74) Mandataire: **Martin, Jean-Jacques et al**
**Cabinet REGIMBEAU 26, Avenue Kléber**
**F-75116 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 089 886 B1

Courier Press, Leamington Spa, England.

**Description**

La présente invention concerne un procédé de préparation d'α-amino-acides libres L par conversion complète de leurs antipodes D pris seuls ou éventuellement en mélanges racémiques.

L'utilisation des α-amino-acides s'est récemment développée de façon importante, en particulier dans les domaines de la médecine et de l'alimentation, et la préparation des α-amino-acides de la série L devient notamment de plus en plus importante.

Trois voies principales sont actuellement connues pour préparer les α-amino-acides L. Il s'agit de:

1. L'utilisation d'hydrolysats de protéines naturelles, dont la phase limitante est la séparation et la purification de chaque acide aminé.

2. La méthode fermentative qui tout en étant plus intéressante que la précédente ne couvre pas la production de tous les α-amino-acides désirés.

3. La synthèse chimique qui peut produire, facilement et à bas prix, de grosses quantités d'α-amino-acides, mais qui a le désavantage de produire essentiellement des mélanges racémiques.

L'intérêt et la rentabilité de cette 3ème voie, certainement la plus porteuse d'avenir du point de vue économique, se trouvent étroitement liés à la disponibilité de méthodes générales de résolution des mélanges racémiques.

Dans ce contexte, l'introduction des enzymes dans la technologie des α-amino-acides constitue actuellement une voie beaucoup plus intéressante que les méthodes de séparation physique ou chimique traditionnelles. Les enzymes actuellement utilisées dans ce but peuvent être des exopeptidases comme par exemple la leucine amino peptidase (LAP) qui, à partir de l'amide d'un α-amino-acide DL n'hydrolyse que l'énantiomère L. (R Koelsch, Enzymologia, *42*, 257 (1972)).

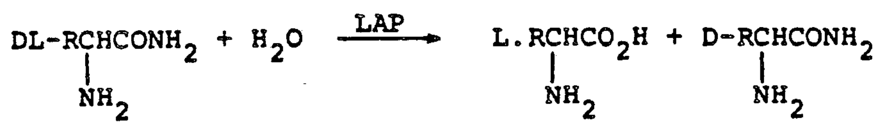


Par un mécanisme analogue, la carboxypeptidase a été utilisée sur les dérivés N chloro acétyl de certains acides aminés (N. Grubhofer and L. Schleith, Naturwissenshaften, *40*, 508 (1953)).

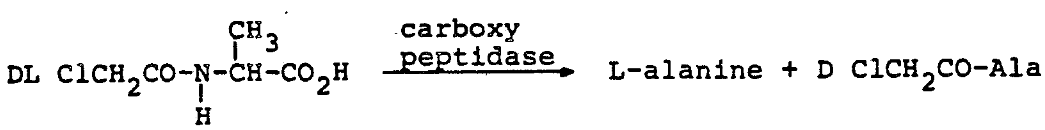


Dans ces deux exemples, la rendement en isomère L ne peut évidemment pas dépasser 50% puisque l'isomère D n'est pas récupéré.

Une amélioration à cette technique enzymatique a été apportée en couplant l'action de l'enzyme a un procédé de racémisation de type chimique (I. Chibata, T. Tosa, T. Sato and T. Mori, Methods in Enzymology, vol. 44, p. 746, Academic Press, 1976) et (I. Chibata, T. Tosa, T. Sato, T. Mori and Y. Matuo, Proceedings of the IVth International Fermentation Symposium: Fermentation Technology Today, p. 383, Society of Fermentation Technology, Japan, 1972).

L'exemple le plus connu concerne l'utilisation, comme enzyme, de l'amino acylase immobilisée qui à partir des N-acétyl acides aminés DL, hydrolyse exclusivement l'isomère L.

L'acide aminé L est alors séparé du milieu par cristallisation après concentration du solvant.

Le D acétyl α-amino-acide restant est soumis alors à un traitement à l'anhydride acétique en milieu anhydre qui conduit à la formation d'une 2 méthyl oxazoline 5 DL. Celle-ci est ensuite hydrolysée en N-acétyl acide aminé DL qui est à nouveau soumis à l'action de l'amino acylase. Par un procédé en continu ce système conduit en conséquence à la récupération totale du mélange racémique sous forme de l'isomère L.

Malgré le progrès considérable amené par ce mode de préparation, il convient cependant de souligner dans ce procédé la très grande différence de concept entre la catalyse enzymatique utilisée et l'étape de racémisation de type chimique non catalytique qui nécessite des conditions expérimentales totalement différentes. En effet, après l'étape d'hydrolyse enzymatique, qui se déroule en solution aqueuse, et après une première concentration qui permet la séparation de l'acide α-aminé L produit, il faut éliminer complètement l'eau qui est alors remplacée par l'anhydride acétique, réactif consommé stoechiométriquement à chaque étape de la racémisation. Puis, avant de revenir à l'étape enzymatique, il faut encore évaporer l'acide acétique formé dans le milieu et le remplacer par l'eau. Ce procédé quoique intéressant est donc très lourd d'un point de vue technique et économique.

La présente invention se rapporte à un procédé de préparation d'α-amino-acides L, par conversion complète de leurs antipodes D soit seuls, soit en mélanges racémiques qui permet précisément d'écarter ces inconvénients. Le procédé conforme à l'invention consiste à introduire, conjointement à une étape de catalyse enzymatique, une étape de racémisation catalytique chimique capable de se produire dans les mêmes conditions réactionnelles (solvants, pH, température) que l'étape de catalyse enzymatique.

Il convient en outre de noter que dans le procédé objet de la présente invention, il n'y a pas de consommation de réactifs dans l'étape de racémisation. De plus, l'utilisation d'un solvant unique permet de concevoir la réalisation d'un système de production en continu extrêmement simplifié et donc très économique.

Le procédé objet de la présente invention est caractérisé en ce que l'on racémise l'antipode D d'un ester dudit α-amino-acide en présence d'un catalyseur chimique constitué par un aldéhyde aromatique de formule générale:

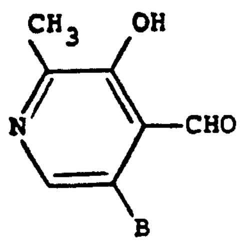

(I)

dans laquelle:

B représente une fonction basique telle qu'une amine tertiaire ou un anion dérivant de l'ionisation d'une fonction acide, tel que $—CO_2^-$, $—OPO_3^-$, $—OPO_3H^-$ et $—SO_3^-$, ledit aldéhyde aromatique étant fixé sur un support insoluble dans le milieu réactionnel et étant introduit dans ledit milieu réactionnel à raison de moins de quatre fois la stoechiométrie par rapport à l'ester de l'α-aminoacide D, et de préférence moins de une fois la stoechiométrie, pour conduire à un mélange en équilibre dynamique des deux formes D et L dudit ester, en ce que l'ester présent sous la forme L est hydrolysé de façon irréversible par voie enzymatique en α-amino-acide L stéréostable correspondant, lesdites étapes de racémisation chimique et d'hydrolyse enzymatique étant conduites dans des conditions réactionnelles identiques, et en particulier dans un solvant choisi parmi des alcools, le formamide, le diméthyl formamide et l'eau, ainsi que les mélanges de ces solvants, et en ce que l'on récupère l'α-amino-acide libre L.

D'autres caractéristiques et avantages de la présente invention apparaîtront à la lecture de la description détaillée faite ci-après.

Il est tout d'abord exposé qu'au cours d'un travail sur les α-amino-acides et certains de leurs dérivés la demanderesse a observé que certains dérivés d'α-amino-acides et particulièrement, les esters optiquement actifs (de configuration D ou de configuration L) se racémisent, lorsqu'ils sont mis en présence de certains composés carbonylés ou d'un mélange de ces composés, et ceci dans divers types de solvants, comme par exemple, les alcools, notamment les alcools aliphatiques inférieurs, tels que le méthanol, l'éthanol, le propanol et l'isopropanol, le formamide, le diméthyl formamide, l'eau ou les mélanges de ces solvants.

Dans l'eau en particulier, et dans des conditions de pH et de températures physiologiques dans lesquelles les enzymes présentent leur activité catalytique maximale, on observe également une excellente racémisation.

Par conditions physiologiques, il convient notamment d'entendre:

— une température comprise entre 0 et 50°C, et plus particulièrement comprise entre 20 et 40°C, et

— un pH compris entre 5 et 10, et plus particulièrement entre 6 et 9.

A titre d'exemples particuliers d'aldéhydes de formule générale I, on mentionnera notamment le pyridoxal-5'-phosphate et le 5'-S-carboxyméthyl thio pyridoxal qui se sont révélés parfaitement appropriés dans leur utilisation comme catalyseurs de racémisation. En solution, ces deux aldéhydes s'ionisent pour donner respectivement les anions suivants:

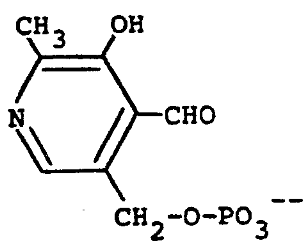

et

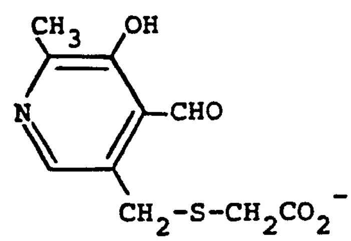

Il convient de noter que parmi les aldéhydes de formule générale I, certains composés comme le pyridoxal-5'-phosphate sont connus, alors que d'autres comme le 5'-S-carboxyméthyl thio pyridoxal sont nouveaux. Ces nouveaux composés peuvent être obtenus par la mise en oeuvre des procédés analogues à celui mentionné ci-après à titre d'exemple pour la préparation du 5'-S-carboxyméthyl thio pyridoxal. Le mode de préparation peut être illustré par le schéma réactionnel suivant.

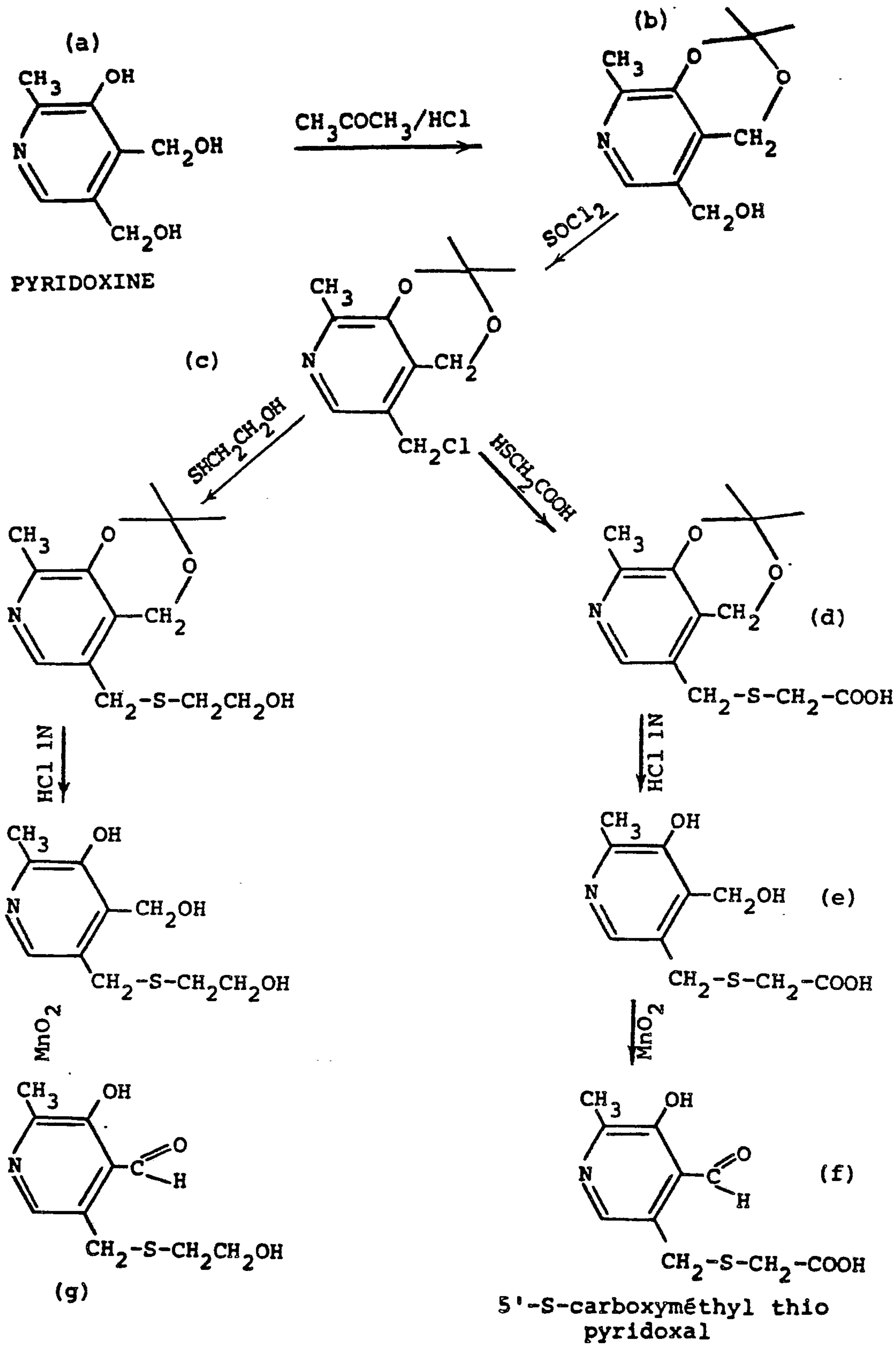


(a) PYRIDOXINE

(b)

(c)

(d)

(e)

(f) 5'-S-carboxyméthyl thio pyridoxal

(g)

Synthèse d'analogues structuraux du pyridoxal

5 g de vitamine $B_6$ (pyridoxine) sous forme de chlorhydrate (a) sont mis en suspension dans 250 ml d'acétone. On envoie dans le mélange de l'acide chlorhydrique gazeux jusqu'à dissolution complète du produit. La solution est laissée 1 heure à température ambiante dans un erlen bouché, après quoi on ajoute 3 volumes d'éther éthylique. Le cétal cyclique (b) sous forme de chlorhydrate précipite immédiatement puis est laissé à cristalliser durant une nuit à 5°C. Le composé (b) est isolé par filtration, lavé à l'éther et séché sous vide à 40° jusqu'à poids constant. Rendement 95°.

1 g du composé (b) est dissous dans 15 ml de $SOCl_2$ et laissé à température ambiante 1 heure.

Le dérivé chloré (c) est récupéré sous forme de poudre blanche après évaporation du solvant et conservé sous vide en présence de soude en pastilles.

Le rendement de cette opération est pratiquement quantitatif.

Le dérivé chloré (c) peut réagir avec différents nucléophiles et constitue donc le produit de départ pour plusieurs synthèses. Il est apparu que les mercaptans sont les plus réactifs parmi les nucléophiles étudiés et le schéma illustre deux exemples de synthèse.

Obtention du 5'-S-carboxyméthyl thio pyridoxal (f)

$10^{-3}$ moles de composé (c) sous forme de chlorhydrate dissous dans 3 ml de méthanol, sont additionnées à $2.10^{-3}$ moles d'acide thio glycolique. On ajoute au mélange 4 ml de méthanol N en soude.

Le NaCl commence à précipiter dans les premières minutes, on laisse la réaction se poursuivre pendant 3 heures à 50°C. Après élimination du NaCl par filtration, le filtrat est concentré à pression réduite et repris par 5 ml d'un mélange $CH_3OH$:HCl (36%) = 8:2. La solution est maintenue à 50°C pendant 3 heures. Le NaCl est éliminé par filtration et le filtrat évaporé à sec à pression réduite. Le produit (e) cristallise sous forme de chlorhydrate par addition d'éther éthylique. Rendement 76%.

Le composé (e) est dissous dans l'eau à une concentration de $10^{-1}$ M et ajusté à pH 7 avec de la soude. Après addition de $MnO_2$ [5 g pour 1 g de produit (e)], on laisse la suspension en agitation vigoureuse 4 à 5 heures à température ambiante. $MnO_2$ est éliminé par centrifugation et le produit (f) peut être isolé après évaporation de l'eau ou bien être utilisé tel quel en solution aqueuse.

L'utilisation du mercaptoéthanol au lieu de l'acide thioglycolique conduit à travers une synthèse analogue au composé (g) qui s'est d'ailleurs avéré très peu actif au cours des expériences de racémisation.

La demanderesse a donc constaté que les aldéhydes aromatiques, analogues structuraux du pyridoxal répondant à la formule générale I, sont utilisables de façon parfaitement satisfaisante en tant que catalyseurs de racémisation, soit en phase homogène, soit en phase hétérogène après immobilisation sur un support insoluble dans le milieu réactionnel. Une telle immobilisation peut être obtenue par toutes les méthodes connues, telles que par liaisons ioniques avec des résines échangeuses d'anions ou par liaisons covalentes qui peuvent être réalisées après une fonctionnalisation adéquate.

La demanderesse a notamment observé que dans des conditions réactionnelles définies précédemment (par exemple en solution aqueuse, entre pH 7 et 8) en présence de pyridoxal 5' phosphate à concentration égale à 0,1 mole par litre, les esters méthyliques des α-amino-acides D ou L sont racémisés avec des temps de 1/2 réaction compris entre 2 et 10 minutes, ceci à une température égale à 20°C et pour une concentration en pyridoxal 5' phosphate sensiblement égale à celle d'ester.

Une telle racémisation a en particulier été obtenue avec les esters méthyliques de l'alanine, l'arginine, l'acide aspartique, l'asparagine, l'acide glutamique, la glutamine, la leucine, la lysine, la β-phényl alanine, la sérine, la tyrosine, le tryptophane, la cystine, la méthionine.

Les esters susceptibles d'être soumis à la première étape de racémisation catalytique chimique sont principalement constitués par les esters alcoyliques, éventuellement activés tels que les thioesters, les α-aminoacides pris soit sous la forme de leurs antipodes D, soit en mélanges racémiques. Ils peuvent être pris sous forme de bases libres ou encore de sels.

Il a également été observé que la vitesse de la racémisation pouvait dépendre de la nature chimique de l'ester; par exemple, elle décroît en passant de l'ester méthylique aux esters éthylique et isopropylique.

La demanderesse a en outre observé que la vitesse de racémisation est également une fonction croissante de la concentration en aldéhyde. Pour une concentration en aldéhyde nettement inférieure à celle de l'ester de l'α-amino-acide, la vitesse de racémisation décroît, mais la totalité de l'ester de l'α-amino-acide est néanmoins racémisée ce qui démontre le pouvoir catalytique de l'aldéhyde dans le processus de racémisation des esters des α-amino-acides.

Il convient également de bien noter que les α-amino-acides libres, contrairement aux esters d'α-amino-acides, ne se racémisent pas en présence de l'aldéhyde, dans les mêmes conditions réactionnelles. Ceci est capital comme on le verra plus tard pour la suite du procédé.

Enfin, il faut veiller à ce que des cations métalliques tels que ceux dérivés par exemple des métaux suivants: Mg, Ca, Bu, Zn, Cd, Hg, Sn, Pb, Mn, Fe, CO, Ni, Cu, Al, ne soient pas présents dans le milieu réactionnel, pour éviter que les α-amino-acides libres puissent être à leur tour racémisés ou desaminés.

A l'issue de ces diverses observations, la demanderesse propose, sans pour autant entendre se limiter à cette interprétation, d'expliquer la racémisation catalytique chimique par la mécanisme suivant:

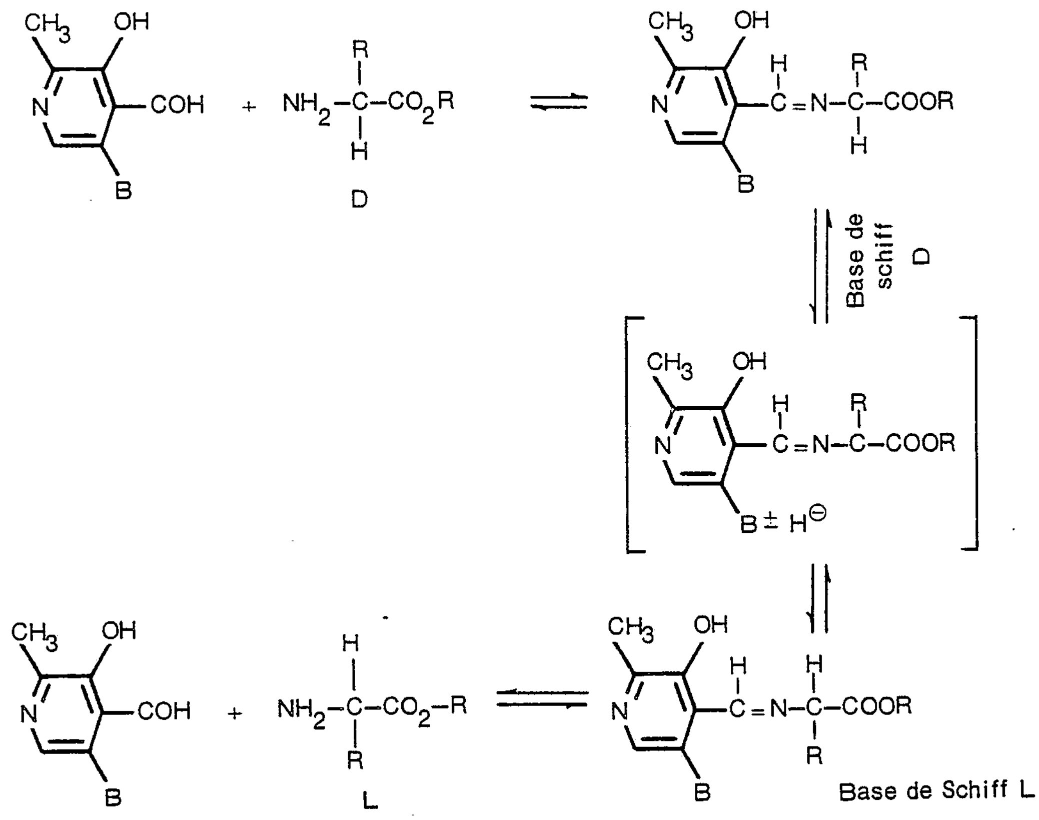


L'intermédiaire envisageable est le passage par un carbanion doublement favorisé, d'une part par sa conjugaison avec la fonction carbonyle de l'ester et la fonction imine, et d'autre part par l'assistance intramoléculaire de la fonction basique (B) qui labilise le proton du carbone asymétrique.

L'importance de cette fonction basique est d'ailleurs mise en lumière par l'observation d'une nette différence d'efficacité catalytique des composés (f) et (g) du schéma de la page 8:

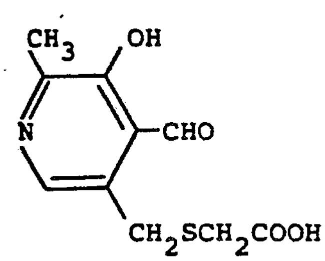

(f)

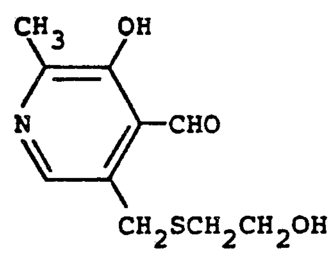

(g)

En effet, le remplacement de la fonction —$COO^-$ par la fonction $CH_2OH$ beaucoup moins basique, fait que le composé (g) devient beaucoup moins actif d'un point de vue catalytique que le composé (f). Ceci illustre bien l'importance de la catalyse bifonctionnelle dans le phénomène de racémisation.

Les aldéhydes ayant une telle structure générale sont considérés comme des catalyseurs bifonctionnels associant la fonction aldéhydique, qui conduit à la base de Schiff de l'acide aminé ester, et la fonction basique responsable de la formation du carbanion, intermédiaire de racémisation.

La catalyse basique qui montre son maximum d'efficacité dans un mécanisme intramoléculaire est cependant également décelable dans un mécanisme intermoléculaire.

La demanderesse a en effet observé que la vitesse de racémisation des acides aminés esters en présence d'une aldéhyde aromatique simple, (par exemple un analogue du pyridoxal exempt de fonction basique) peut être sensiblement augmentée par la présence dans le milieu réactionnel d'une fonction basique, telle qu'un anion carboxylate (acétate de sodium ou acétate de trialkyl ammonium). Les conditions de température et de pH restant constantes au cours de ces expériences.

On notera enfin que dans le cas des α-amino-acides libres, l'absence de la fonction ester est suffisante pour empêcher tout arrachement du proton lié au carbone assymétrique.

Selon ce schéma, une partie de l'ester de l'α-amino-acide, est bloquée sous forme de base de Schiff dans une proportion qui est fonction de son rapport de concentration avec le catalyseur.

D'un point de vue économique, le rapport entre catalyseur et ester devra être le résultat d'un

compromis entre une vitesse satisfaisante de racémisation (d'autant plus grande qu'une plus grande concentration d'ester est piégés sous forme de base de Schiff) et la nécessité d'avoir une concentration importante d'ester libre présent à l'équilibre afin que celui-ci puisse (comme on le verra plus tard) être soumis à l'hydrolyse enzymatique.

Ce processus de racémisation ayant été observé et expliqué, on remarque que les esters des α-amino-acides concernés peuvent être considérés comme des substrats pour certaines enzymes.

De plus, les conditions dans lesquelles s'opèrent les racémisations coïncident rigoureusement avec celles requises en général pour le fonctionnement des enzymes.

Il est connu en effet qu'un certain nombre de dérivés carboxyliques des α-amino-acides, parmi lesquels les esters peuvent être hydrolysés en α-amino-acides libres par catalyse enzymatique. Certaines de ces enzymes sont dotées d'une haute chiralité et n'hydrolysent que les isomères L en laissant inaltérés les antipodes D présents.

La demanderesse a observé qu'une telle catalyse enzymatique, couplée à la catalyse de racémisation ainsi qu'elle a été définie précédemment, appliquée à des esters d'α-amino-acides, soit de configuration D, soit de configuration L, soit du mélange des deux antipodes dans n'importe quel rapport, conduit dans tous les cas à la seule obtention de l'α-amino-acide libre de la série L avec un rendement quantitatif par rapport à l'ester de l'α-amino-acide de départ.

Le rendement quantitatif de ce procédé est expliqué par le fait que le substrat de la réaction enzymatique est en équilibre de racémisation, alors que le produit de la réaction, qui se forme de manière irréversible, est stéréostable dans les mêmes conditions et peut donc s'accumuler.

Le principe de fonctionnement global du système peut être alors schématisé ainsi:

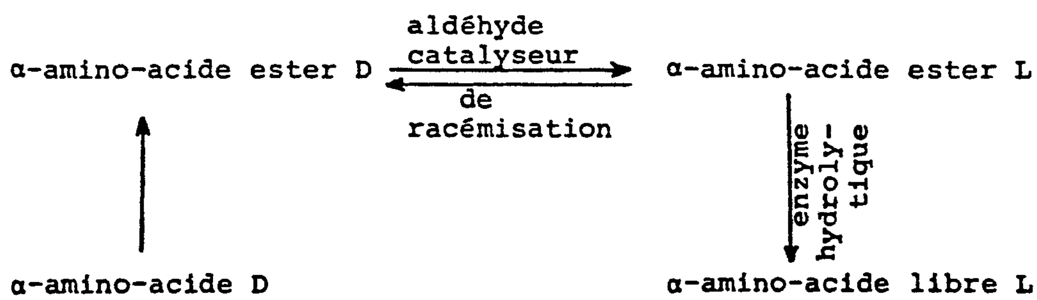


Dans ce schéma général illustrant le redressement de configuration, la première étape est l'estérification des α-amino-acides D, L ou DL, pour laquelle on dispose d'un certain nombre de réactifs déjà connus, tels que par exemple l'alcool additionné d'un catalyseur acide, ou du méthylsulfate ou du diméthyl sulfite.

A partir des esters, les deux types de catalyse impliquées: la catalyse de racémisation, qui produit les substrats pour l'enzyme, et la catalyse enzymatique qui suit, peuvent évidemment agir ensemble dans un système homogène. Néanmoins, ce mode d'action tout en étant le plus simple à réaliser comporte un certain nombre d'inconvénients dont les plus importants sont la séparation des α-amino-acides libres et la récupération des catalyseurs.

De plus, la réaction chimique possible entre le(s) catalyseur(s) de racémisation et certains groupements fonctionnels des enzymes peuvent conduire à une perte d'activité des catalyseurs, mais également à une modification de l'activité catalytique de l'enzyme.

Pour ces raisons, il est préférable de faire agir ces deux catalyseurs en les maintenant séparés dans le milieu réactionnel par immobilisation, par exemple par fixation sur des supports insolubles dans le milieu réactionnel.

Les supports insolubles portant les deux catalyseurs peuvent être utilisés comme matériel de remplissage de deux réacteurs séparés et reliés par un système de circulation du milieu liquide.

Un système plus simplifié consiste à mélanger aux milieux réactionnels les deux supports insolubles portant les deux catalyseurs dans un seul réacteur.

Etant donné les caractères consécutifs de ces deux types de catalyse, un modèle de réacteur envisageable pourrait être également constitué par un ensemble de couches des deux catalyseurs immobilisées et empilées de manière alternative dans un réacteur à circulation.

Les enzymes immobilisées le seront par toutes les méthodes de l'art, de même que les catalyseurs aldéhydiques de racémisation.

Les supports utilisés lors de la mise en oeuvre du procédé de l'invention, pour fixer le catalyseur aldéhydique de racémisation, ont été des résines échangeuses d'anions telles que la diéthylaminoéthyl Sephadex®, la diéthylaminoéthyl cellulose, ou des résines à matrices polystyréniques portant des ammonium quaternaires. Sur de telles résines, le pyridoxal phosphate se fixe par exemple complètement de façon ionique dans l'intervalle de pH utilisé et défini précédemment.

Les enzymes utilisées dans ce procédé l'ont été soit en phase homogène, soit fixées sur supports insolubles dans le milieu réactionnel, à matrice polyacrylique ou polysaccharidique.

Dans ces réactions, les supports insolubles et les modes de fixation sont donnés à titre d'exemples.

Les enzymes les plus courantes utilisables dans le procédé de l'invention, sont les enzymes hydrolytiques telles que trypsine, α-chymotrypsine, papaïne, chymopapaïne, leucine amino peptidase, cette liste n'étant nullement limitative. Leur choix particulier est fonction de leur spécificité vis-à-vis de l'α-amino-acide à préparer.

Si le but essentiel de ce procédé est la production d'acides alpha-aminés L à partir de mélanges racémiques, il peut aussi permettre, à partir d'acides alpha-aminés optiquement actifs ou non, la substitution du proton porté par le carbone alpha par un deutérium ou un tritium en travaillant dans l'eau lourde ou l'eau tritiée.

Les exemples suivants illustrent le procédé de l'invention.

Exemples de racémisation en phase homogène:

Dans une expérience type, à une solution aqueuse $10^{-1}$ molaire d'un ester d'α-amino-acide L ou D sous forme de chlorhydrate, ramené à pH 7 par addition de soude, on ajoute un volume égal d'une solution aqueuse de pyridoxal 5' phosphate (pH 7) de molarité inférieure ou égale à celle de l'ester d'α-amino-acide. La température est maintenue constante à la valeur désirée, par exemple à 20°C.

Le pouvoir rotatoire de la solution est suivi en continu à 546 nanomètre à l'aide d'un micro-polarimètre. Lorsque ce pouvoir rotatoire est nul (dans des temps de 1/2 réaction variables fonctions de la température et du rapport de concentration ester/pyridoxal) le mélange réactionnel est analysé par chromatographie en couche mince et comparé à la solution de départ pour contrôler que la fonction ester n'a pas été hydrolysée chimiquement.

Les résultats concernant un certain nombre d'α-amino-acides sont donnés dans le tableau ci-après:

| Exemples n° | esters méthyliques de | $R = \frac{[\text{pyridoxal 5' phosphate}]}{[\text{ester d'}\alpha\text{-amino-acide}]} = 1$ t 1/2 (min) de racémisation | $R = \frac{[\text{pyridoxal 5' phosphate}]}{[\text{ester d'}\alpha\text{-amino-acide}]} = 0,5$ t 1/2 (min) de racémisation |
|---|---|---|---|
| 1 | Alanine | 3,9 | 5,1 |
| 2 | Leucine | 6,6 | 14 |
| 3 | β-phénylalanine | 3,8 | 9,6 |
| 4 | Tyrosine | 12,1 | 30,39 |
| 5 | Méthionine | 16,7 | 10,5 |
| 6 | Lysine | 4,62 | 13,4 |
| 7 | Glutamique | 8,1 | 17,9 |
| 8 | Aspartique | 4,2 | 10,4 |
| 9 | Leucine éthyl ester | 9,02 | |
| 10 | Leucine isopropyl ester | 10,07 | |

Exemples de racémisation en phase hétérogène et résolution enzymatique du mélange racémique:

Example 11

Dans une expérience type, le pyridoxal 5' phosphate en solution $0,510^{-2}$ molaire dans l'eau est ramené à pH 7, ce qui constitue une solution mère.

50 microlitres de cette solution sont dilués dans 3 ml de tampon acétate (pH 5,4) et la densité optique est mesurée à 400 nm pour contrôle. On ajoute alors à 20 ml de la solution mère, 3 g de résine diéthylaminoéthyl cellulose et la suspension est maintenue à pH 7 sous agitation.

Après 5 minutes on laisse décanter et prélève 50 microlitres de surnageant qui est analysé spectralement à 400 nm dans les conditions précédentes. La densité optique nulle observée indique que le pyridoxal 5' phosphate est complètement fixé au support. La D tyrosine méthyl ester est alors ajoutée à la solution ainsi obtenue pour avoir une concentration finale de $10^{-2}$ molaire. La suspension est maintenue sous agitation vigoureuse à température constante égale à 20°C.

A des intervalles de temps régulier, des séries de trois échantillons sont alors prélevés:

— le premier échantillon est analysé par analyse automatique et permet de montrer qu'il n'y a pas trace d'α-amino-acide libre dans le milieu.

— le deuxième échantillon après dilution convenable, est analysé spectrophotométriquement (λ max. 274 nm) et permet de déterminer la concentration de tyrosine méthyl ester libre qui est le seul chromophore présent dans le surnageant (la partie manquante restant accrochée au catalyseur).

— le troisième échantillon est soumis à hydrolyse chymotrypsique, qui ne libère que l'α-amino-acide L à partir de l'ester correspondant.

Les résultats obtenus sont indiqués dans le tableau suivant:

| t min | tyrosine ester fixée en% | tyrosine ester libre en% | racémisation en % | L tyrosine libérée |
|---|---|---|---|---|
| 5 | 0,49 | 0,51 | 14 | 7 |
| 10 | " | " | 16 | 8 |
| 20 | " | " | 47 | 23,5 |
| 40 | " | " | 75 | 37,5 |
| 80 | " | " | 93 | 46,5 |
| 160 | " | " | 100 | 50 |

Exemple 12

En utilisant le même protocole expérimental que dans l'exemple 11, mais à une température de 40°C, on obtient les résultats suivants:

| t min | tyrosine méthyl ester fixée en % | tyrosine méthyl ester libre en % | racémisation en % | L tyrosine libérée % |
|---|---|---|---|---|
| 5 | 0,49 | 0,51 | 95 | 47,5 |
| 10 | " | " | 100 | 50 |
| 80 | " | " | 100 | 50 |

Exemple 13

5'-S-carboxyméthyl thio pyridoxal: racémisation en phase homogène et résolution enzymatique du mélange racémique.

5 ml d'une solution aqueuse de 5'-S-carboxyméthyl thio pyridoxal $10^{-2}$ M ramené à pH 7, sont additionnés à 5 ml d'une solution aqueuse de D-phénylalanine méthyl ester $5 \times 10^{-2}$ M à pH 7. Le mélange est maintenu sous agitation à une température de 20°C. Aux temps voulus (voir tableau) 1 ml du mélange réactionnel est dilué avec 1 ml de $NaHCO_3$ 5% dans l'eau et extrait par 2 ml d'acétate d'éthyle saturés d'eau. Ceci permet une séparation nette du catalyseur de racémisation qui reste dans la phase aqueuse, alors que la phase organique contiendra l'ester de l'α-amino-acide.

Une fraction de la phase organique est évaporée, soumise à une hydrolyse acide et analysée sur analyseur automatique d'α-amino-acides. Cette opération permet de déterminer quantitativement l'ester d'α-amino-acide séparé par extraction.

Une autre fraction est évaporée et traitée avec l'α-chymotrypsine: la phénylalanine libérée est analysée sur analyseur automatique. Cette dernière analyse permet de déterminer la fraction d'isomère L présent dans le mélange (seul à être digéré).

Des contrôles chromatographiques ont montré l'absence de phénylalanine libre dans la phase acétate d'éthyle de départ.

Les résultats obtenus sont résumés dans le tableau ci-après.

| temps de racémisation (min) | L-phénylalanine formée (%) | taux de racémisation (%) |
|---|---|---|
| 20 | 25 | 50 |
| 80 | 36 | 72 |

Exemples de redressement de configuration:

Exemple 14

Le pyridoxal 5' phosphate est immobilisé comme décrit dans les exemples 11 et 12 à une concentration identique à celle décrite dans l'exemple 11 (20 ml de solution $0{,}510^{-2}$ molaire plus 3 g de résine diéthylaminoéthyl cellulose, c'est-à-dire 0,1 milliéquivalent).

On ajoute à cette solution 50,9 mg de D tryptophane méthyl ester sous forme de chlorhydrate (0,2 milliéquivalent). Le pH est ajusté à 7, et la température maintenue à 40°C.

20 mg de chymotrypsine sont alors ajoutés à cette solution.

Après 30 minutes de réaction le pyridoxal phosphate fixé sur support est séparé du milieu par filtration. Le volume du surnageant est mesuré, égal à 17 ml.

Une partie aliquote est soumise dans un premier temps à deux analyses:

1. Il est montré par chromatographie la disparition complète de l'α-amino-acide ester, et la seule présence dans la phase liquide de l'α-amino-acide libre.

2. Une deuxième analyse quantitative sur auto-analyseur indique que la quantité de tryptophane libre présent dans le surnageant séparé est égale à 40% de la quantité molaire d'ester initialement introduit dans le milieu (les 60% restant sont considérés fixés sur le support directement et par l'intermédiaire du catalyseur ainsi que dans la phase liquide baignant le support).

A la phase solide contenant le catalyseur de racémisation, on ajoute alors 17 ml d'eau et 16,5 mg de D tryptophane méthyl ester (équivalent à la quantité de tryptophane précédemment hydrolysée) plus 10 mg de chymotrypsine.

Après de nouveau temps réactionnel de 30 minutes à 40°C on sépare à nouveau les deux phases. Le tryptophane acide libre est dosé. Le taux de transformation de cette réaction:

D tryptophane ester → acide libre L

est quantitatif si on le compare à la dernière addition.

Le surnageant est lyophilisé et l'α-amino-acide libre purifié par filtration sur Sephadex® G 25 équilibré avec de l'acide acétique à 1%.

La fraction contenant l'α-amino-acide est lyophilisée à nouveau et l'α-amino-acide est estérifié par la méthode méthanol-chlorure de thionyle à température ambiante.

L'ester ainsi obtenu est soumis à nouveau à l'hydrolyse chymotrypsique en l'absence de pyridoxal phosphate. La totalité de l'ester est rapidement hydrolysée en α-amino-acide libre, ce qui démontre son appartenance à la série L.

Des essais parallèles sur des esters d'α-amino-acides D n'ont donné dans des conditions comparatives aucune trace d'α-amino-acide libre.

Exemple 15

La D tyrosine méthyl ester a été traitée comme le D tryptophane de l'exemple 14.

La récupération de la L tyrosine après la première étape a été de 42%.

Le remplacement de cette première fraction de L tyrosine par une quantité équivalente de D tyrosine ester sur le même catalyseur, a conduit, dans les mêmes conditions opératoires que celles de l'exemple 14, à une conversion quantitative en L tyrosine.

La série stérique de l'α-amino-acide a été contrôlée comme précédemment.

Exemple 16

La D phénylalanine méthyl ester dans les mêmes conditions opératoires que l'exemple 14 donne 39% de L phénylalanine à la première addition (61% restant réversiblement fixés sur le support).

La deuxième addition est transformée quantitativement ainsi qu'un troisième. Ceci montre la possibilité de travail en continu de ce système.

Bien entendu, la présente invention ne saurait être limitée aux exemples de mise en oeuvre particuliers décrits. Il est en revanche parfaitement possible, sans pour autant sortir du cadre de la présente invention, d'en imaginer un certain nombre de variantes, en particulier dans le choix de l'ester soumis à la racémisation. Le procédé de la présente invention permet de façon très générale d'assurer le redressement de configuration d'antipodes D de divers α-amino-acides, tels que l'alanine, la valine, la leucine, l'isoleucine, la β-phénylalanine, la sérine, la thréonine, la lysine, la δ-hydroxylysine, l'arginine, l'acide aspartique, l'asparagine, l'acide glutamique, la glutamine, la cystéine, la cystine, la méthionine, la tyrosine, la thyroxine, la proline, l'hydroxyproline, le tryptophane et l'histidine.

**Revendications**

1. Procédé de préparation d'un α-amino-acide libre L, caractérisé en ce que l'on racémise l'antipode D d'un ester dudit α-amino-acide en présence d'un catalyseur chimique constitué par un aldéhyde aromatique de formule générale:

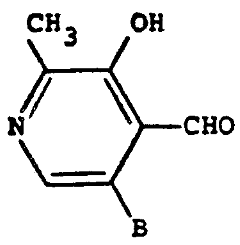

(I)

dans laquelle:

B représente une fonction basique telle qu'une amine tertiaire ou un anion dérivant de l'ionisation d'une fonction acide, tel que $—CO_2^-$, $—OPO_3^-$, $—OPO_3H^-$ et $—SO_3^-$, ledit aldéhyde aromatique étant fixé sur un support insoluble dans le milieu réactionnel et étant introduit dans ledit milieu réactionnel à raison de moins de quatre fois la stoechiométrie par rapport à l'ester de l'α-aminoacide D, et de préférence moins de une fois la stoechiométrie, pour conduire à un mélange en équilibre dynamique des deux formes D et L dudit ester, en ce que l'ester présent sous la forme L est hydrolysé de façon irréversible par voie enzymatique en α-amino-acide L stéréostable correspondant, lesdites étapes de racémisation chimique et d'hydrolyse enzymatique étant conduites dans des conditions réactionnelles identiques, et en particulier dans un solvant choisi parmi des alcools, le formamide, le diméthyl formamide et l'eau, ainsi que les mélanges de ces solvants, et en ce que l'on récupère l'α-amino-acide libre L.

2. Procédé selon la revendication 1, caractérisé en ce que ledit aldéhyde aromatique répond à la formule I dans laquelle la fonction basique B représente le groupement $—CH_2—O—PO_3^-$.

3. Procédé selon la revendication 1, caractérisé en ce que ledit aldéhyde aromatique répond à la formule I dans laquelle la fonction basique B représente le groupement $—CH_2—S—CH_2—CO_2^-$.

4. Procédé selon l'une des revendications 1 à 3, caractérisé en ce que les étapes de racémisation chimique et d'hydrolyse enzymatique sont conduites dans un milieu réactionnel dont le pH est sensiblement compris entre 5 et 10 et plus particulièrement entre 6 et 9.

5. Procédé selon l'une des revendications 1 à 4, caractérisé en ce que les étapes de racémisation chimique et d'hydrolyse enzymatique sont conduites dans un milieu réactionnel dont la température est sensiblement comprise entre 0 et 50°C et plus particulièrement entre 20 et 40°C.

6. Procédé selon l'une des revendications 1 à 5, caractérisé en ce que l'ester de départ de l'α-amino-acide est introduit dans le milieu réactionnel sous la forme d'un mélange racémique.

7. Procédé selon l'une des revendications 1 à 6, caractérisé en ce que l'ester de départ de l'α-amino-acide utilisé est choisi parmi les esters méthylique, éthylique, propylique ou isopropylique, sous la forme d'une base libre ou sous forme d'un sel.

8. Procédé selon l'une des revendications 1 à 7, caractérisé en ce que l'étape d'hydrolyse enzymatique est obtenue à l'aide d'estérases telles que la trypsine, la chymotrypsine, la papaïne, la chymopapaïne et la leucine amino peptidase.

9. Procédé selon la revendication 8, caractérisé en ce que lesdites estérases sont fixés sur un support insoluble dans le milieu réactionnel.

10. Procédé selon l'une des revendications 1 à 9, caractérisé en ce que l'ester de départ de l'α-amino-acide D est obtenu, par des moyens en soi connus, à partir de l'α-amino-acide D.

11. Procédé selon l'une des revendications 1 à 10, caractérisé en ce que l'on prépare l'antipode L d'un α-amino-acide choisi parmi: l'alanine, la valine, la leucine, l'isoleucine, la β-phénylalanine, la sérine, la thréonine, la lysine, la δ-hydroxylysine, l'arginine, l'acide aspartique, l'asparagine, l'acide glutamique, la glutamine, la cystéine, la cystine, la méthionine, la tyrosine, la thyroxine, la proline, l'hydroxyproline, le tryptophane et l'histidine.

12. Le 5'-S-carboxyméthyl-thio-pyridoxal de formule

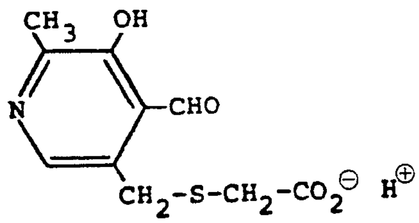

en tant que catalyseur chimique de racémisation nécessaire pour la mise en oeuvre du procédé selon l'une des revendications 1 à 11.

**Patentansprüche**

1. Verfahren zur Herstellung einer freien L-α-Aminosäure, dadurch gekennzeichnet, daß man den D-Antipoden eines Esters dieser α-Aminosäure racemisiert, in Anwesenheit eines chemischen Katalysators, bestehend aus einem aromatischen Aldehyd der allgemeinen Formel

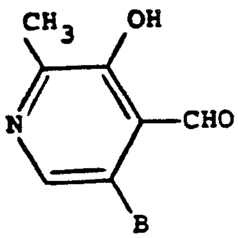

(I)

worin

B eine basische Funktion, wie ein tertiäres Amin oder ein Anion darstellt, das von der Ionisierung einer Säurefunktion stammt, wie $—CO_2^-$, $—OPO_3^-$, $—OPO_3H^-$ und $—SO_3^-$, wobei der aromatische Aldehyd auf einen in dem Reaktionsmedium unlöslichen Träger fixiert ist und in das Reaktionsmedium in einer Menge von weniger als dem 4-fachen der Stöchiometrie, bezogen auf den D-α-Aminosäureester und vorzugsweise von weniger als einmal der Stöchiometrie eingebracht wird, um zu einem dynamischen Gleichgewichts-Gemisch der beiden Formen D und L des Esters zu führen, und daß der in der L-Form vorliegende Ester in irreversibler Form auf enzymatischem Wege zur entsprechenden stereostabilen L-α-Aminosäure hydrolysiert wird, wobei die chemischen Racemisierungs- und enzymatischen Hydrolysierungsstufen unter identischen Reaktionsbedingungen durchgeführt werden, und insbesondere in einem Lösungsmittel, ausgewählt aus den Alkoholen, Formamid, Dimethylformamid und Wasser, sowie den Gemischen dieser Lösungsmittel, und daß man die freie L-α-Aminosäure gewinnt.

2. Verfahren nach Anspruch 1, dadurch gekennzeichnet, daß der aromatische Aldehyd der Formel I entspricht, worin die basische Funktion B die Gruppe $—CH_2—O—PO_3^-$ darstellt.

3. Verfahren nach Anspruch 1, dadurch gekennzeichnet, daß der aromatische Aldehyd der Formel I entspricht, worin die basische Funktion B die Gruppe $—CH_2—S—CH_2—CO_2^-$ darstellt.

4. Verfahren nach einem der Ansprüche 1 bis 3, dadurch gekennzeichnet, daß die Stufen der chemischen Racemisierung und der enzymatischen Hydrolyse in einem Reaktionsmedium durchgeführt werden, dessen pH genau bei 5 bis 10 und insbesondere bei 6 bis 9 liegt.

5. Verfahren nach einem der Ansprüche 1 bis 4, dadurch gekennzeichnet, daß die Stufen der chemischen Racemisierung und der enzymatischen Hydrolyse in einem Reaktionsmedium durchgeführt werden, dessen Temperatur genau bei 0 bis 50°C und insbesondere bei 20 bis 40°C liegt.

6. Verfahren nach einem der Ansprüche 1 bis 5, dadurch gekennzeichnet, daß der Ausgangsester der α-Aminosäure in das Reaktionsmedium in der Form eines racemischen Gemisches eingeführt wird.

7. Verfahren nach einem der Ansprüche 1 bis 6, dadurch gekennzeichnet, daß der verwendete Ausgangsester der α-Aminosäure ausgewählt wird aus den Methyl-, Ethyl-, Propyl- oder Isopropylestern, in der Form einer freien Base oder in der Form eines Salzes.

8. Verfahren nach einem der Ansprüche 1 bis 7, dadurch gekennzeichnet, daß die Stufe der enzymatischen Hydrolyse mit Hilfe von Esterasen, wie Trypsin-, Chymotrypsin-, Papain-, Chymopapain- und Leucin-aminopeptidase durchgeführt wird.

9. Verfahren nach Anspruch 8, dadurch gekennzeichnet, daß die Esterasen an einem in dem Reaktionsmedium unlöslichen Träger fixiert sind.

10. Verfahren nach einem der Ansprüche 1 bis 9, dadurch gekennzeichnet, daß der Ausgangsester der D-α-Aminosäure in ansich bekannter Weise ausgehend von der D-α-Aminosäure erhalten wird.

11. Verfahren nach einem der Ansprüche 1 bis 10, dadurch gekennzeichnet, daß man den L-Antipoden einer Aminosäure herstellt ausgewählt aus Alanin, Valin, Leucin, Isoleucin, β-Phenylalanin, Serin, Threonin, Lysin, δ-Hydroxylysin, Arginin, Asparaginsäure, Asparagin, Glutaminsäure, Glutamin, Cystein, Cystin, Methionin, Tyrosin, Thyroxin, Prolin, Hydroxyprolin, Tryptophan und Histidin.

12. 5'-S-Carboxymethyl-thio-pyridoxal der Formel

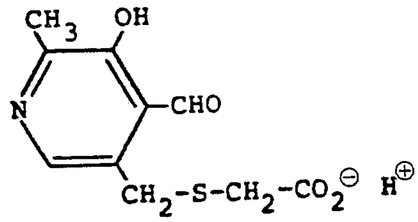

als chemischer Racemisierungskatalysator, benötigt zur Durchführung des Verfahrens nach einem der Ansprüche 1 bis 11.

**Claims**

1. A method of preparing a free L α-amino acid, characterised in that the D antipode of an ester of the α-amino acid is racemised in the presence of a chemical catalyst comprising an aromatic aldehyde having the general formula:

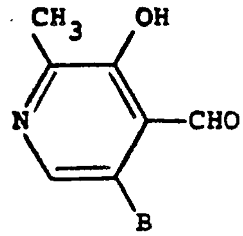

(I)

in which:

B represents a basic group such as a tertiary amine or an anion obtained by ionization of an acid group such as $—CO_2^-$, $—OPO_3^-$, $—OPO_3H^-$ and $—SO_3^-$, the aromatic aldehyde being fixed to a support which is insoluble in the reaction medium and being introduced into the reaction medium in the proportion of less than 4 times the stoichiometry relative to the ester of the D α-amino acid and preferably less than once times the stoichiometry, to obtain a mixture of the two D and L forms of the ester in equilibrium, and in that the ester present in L form is irreversibly hydrolyzed by enzymatic action into the corresponding stereostable L α-amino acid, the chemical racemisation and enzymatic hydrolysis steps being carried out under identical reaction conditions, more particularly in a solvent chosen from among alcohols, formamides, dimethyl formamide and water or mixtures of these solvents, and the free L α-amino acid recovered.

2. A method according to claim 1, characterised in that the aromatic aldehyde has the formula I in which the basic group B represents the group $—CH_2—O—PO_3^-$.

3. A method according to claim 1, characterised in that the aromatic aldehyde has the formula I in which the basic group B represents the group $—CH_2—S—CH_2—CO_2^-$.

4. A method according to any of claims 1 to 3, characterised in that the chemical racemisation and enzymatic hydrolysis steps are carried out in a reaction medium at a pH substantially between 5 and 10 and more particularly between 6 and 9.

5. A method according to any of claims 1 to 4, characterised in that the chemical racemisation and enzymatic hydrolysis steps are carried out in a reaction medium at a temperature substantially between 0 and 50°C and more particularly between 20 and 40°C.

6. A method according to any of claims 1 to 5, characterised in that the starting α-amino acid ester is introduced into the reaction medium in the form of a racemic mixture.

7. A method according to any of claims 1 to 6, characterised in that the starting ester of the α-amino acid used is chosen from among the methyl, ethyl, propyl or isopropyl esters, in the form of a free base or in the form of a salt.

8. A method according to any of claims 1 to 7, characterised in that the enzymatic hydrolysis step is brought about by use of esterases such as trypsin, chymotrypsin, papin, chymopapain and leucine amino peptidase.

9. A method according to claim 8, characterised in that the esterases are fixed on a support which is insoluble in the reaction medium.

10. A method according to any of claims 1 to 9, characterised in that the starting ester of the D α-amino acid is obtained by known means from the D α-amino acid.

11. A method according to any of claims 1 to 10, characterised by preparation of the L antipode of an α-amino acid chosen from among alanine, valine, leucine, isoleucine, β-phenylalanine, serine, threonine, lysine, δ-hydroxylysine, arginine, aspartic acid, asparagine, glutamic acid, glutamine, cysteine, cystine, methionine, tyrosine, thyroxine, proline, hydroxyproline, tryptophan and histidine.

12. 5'-S-carboxymethyl-thio-pyridoxal having the formula

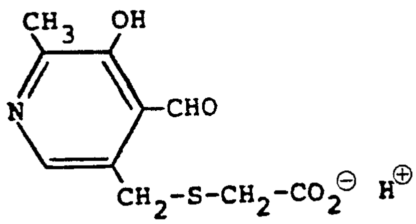

as a chemical racemisation catalyst necessary for working the method according to any of claims 1 to 11.